# FASCICULE DE BREVET EUROPEEN

(11) **EP 1 367 954 B1**
(45) Date de publication et mention de la délivrance du brevet: **05.01.2005**
(21) Numéro de dépôt: 02722342.9
(22) Date de dépôt: 13.03.2002
(51) Int. Cl.: A61B 17/70

(54) **IMPLANT INTERVERTEBRAL A FIXATION AUTO-BLOQUANTE**
ZWISCHENWIRBELIMPLANTAT MIT SELBSTBLOCKIERENDEM BEFESTIGUNGSMITTEL
intervertebral implant with self-locking fixation

(30) Priorité: 13.03.2001 FR 0103362
(43) Date de publication de la demande: 10.12.2003
(73) Titulaire: Spine Next, La Cité Mondiale, 33000 Bordeaux (FR)
(72) Inventeur: PASQUET, Denis, F-33600 Pessac (FR); LE COUEDIC, Régis, F-33000 Bordeaux (FR); SENEGAS, Jacques, F-33700 Merignac (FR)
(74) Mandataire: Dronne, Guy
(86) Numéro de dépôt international: PCT/FR2002/000888
(87) Numéro de publication internationale: WO 2002/071960

(56) Documents cités:
- FR-A- 2 704 745
- FR-A- 2 722 088
- FR-A- 2 775 183
- US-A- 5 496 318

## Description

La présente invention concerne un implant intervertébral comprenant une cale dans laquelle sont ménagées deux gorges opposées l'une de l'autre que l'axe longitudinal de ladite cale traverse et qui sont susceptibles de recevoir les deux apophyses épineuses de deux vertèbres. La cale présente deux parois latérales et au moins un lien de fixation pour maintenir lesdites apophyses épineuses dans lesdites gorges.

Des implants intervertébraux comprenant une cale destinée à être insérée entre les apophyses épineuses, qui prolongent la partie postérieure des vertèbres, pour en limiter le rapprochement sont bien connus. Le document FR 2,775,183 sur lequel est basé le préambule de la revendication 1 montre un tel implant. L'installation de tels implants au niveau du rachis nécessite une intervention chirurgicale lourde durant laquelle on désinsère le ligament interépineux reliant les deux vertèbres entre lesquelles on veut interposer la cale et on ménage des évidements dans l'espace interépineux supérieur et inférieur sous les ligaments interépineux de façon à introduire les liens. Une première extrémité du lien est susceptible d'être relié à ladite cale avant qu'elle soit interposée entre les apophyses épineuses, par exemple à au moins une des ailes de ladite gorge, puis le lien est introduit dans l'évidement interépineux de façon à entourer l'apophyse épineuse pour être ensuite relié à l'autre aile de la gorge. Ainsi, l'apophyse épineuse est maintenue par le lien dans la gorge de la cale.

Un seul lien est susceptible d'être utilisé pour maintenir la cale et alors il se prolonge sur la paroi latérale de la cale pour rejoindre l'une des ailes de la gorge opposée puis entourer l'autre apophyse épineuse pour être relié à l'autre aile de la gorge opposée.

Alors que l'interposition de la cale entre les deux apophyses épineuses est relativement aisée et rapide, le rattachement des liens sur la cale est lui, beaucoup plus délicat compte tenu de l'encombrement de l'espace interépineux. Ainsi, la réalisation de boucles et de noeuds est relativement longue et représente une partie non négligeable du temps opératoire.

Un objet de la présente invention est de réaliser un implant intervertébral comprenant une cale et au moins un lien qui permettent un montage aisé et rapide dudit implant.

Dans ce but, la présente invention présente un implant comprenant, au moins une pièce de fixation auto-bloquante amovible présentant des premiers moyens de liaison et à travers laquelle ledit lien est susceptible de coulisser lorsqu'il est entraîné en translation selon une première direction, ladite pièce de fixation auto-bloquante étant susceptible de bloquer ledit lien en translation selon une seconde direction opposée à ladite première direction ; et au moins une des deux parois latérales de ladite cale comporte des deuxièmes moyens de liaison aptes à coopérer avec lesdits premiers moyens de liaison pour relier ladite pièce de fixation auto-bloquante amovible à la paroi latérale de ladite cale, l'entraînement de ladite extrémité libre dudit lien de façon à entraîner ledit lien en translation selon ladite première direction produisant le serrage de ladite apophyse épineuse dans ladite gorge et le blocage dudit lien en translation selon ladite seconde direction par rapport à ladite cale.

Ainsi, une caractéristique de l'implant conforme à l'invention, réside dans le mode de rattachement dudit lien à ladite cale au moyen de la pièce de fixation auto-bloquante amovible qui présente des premiers moyens de liaison et qui est susceptible d'être reliée à ladite cale munie de deuxièmes moyens de liaison, après que cette dernière a été interposée entre les deux apophyses épineuses. De la sorte, ledit lien dont la première extrémité est reliée à la cale est introduit dans l'évidement interépineux supérieur ou inférieur à l'espace interépineux dans lequel la cale est interposé, puis la seconde extrémité libre dudit lien est inséré dans ladite pièce de fixation auto-bloquante amovible en dehors du corps de l'individu qui subit l'intervention. La pièce de fixation auto-bloquante amovible est alors portée au regard de la paroi latérale de la cale tout en faisant coulisser le lien à travers ladite pièce qui est ensuite reliée à la cale par la coopération des premiers et deuxièmes moyens de liaison. Le serrage du lien qui entoure l'apophyse épineuse et qui la maintient dans la gorge de la cale est achevé après que la pièce de fixation auto-bloquante amovible a été reliée à la cale. Lorsque la seconde extrémité libre du lien est entraînée en translation selon la première direction, la tension du lien dans la pièce de fixation auto-bloquante amovible induit une pression apte à bloquer ledit lien en translation selon une deuxième direction inverse à la première direction.

Selon un mode particulier de mise en oeuvre de l'invention, ladite pièce de fixation auto-bloquante amovible présente une première face principale opposée à une seconde face principale qui sont reliées aux deux extrémités de ladite pièce de fixation auto-bloquante amovible et un évidement central formant fente, débouchant dans lesdites faces principales que ledit lien traverse, de façon qu'une première portion dudit lien comprise entre ladite première extrémité et ledit évidement central soit susceptible de comprimer une deuxième portion dudit lien comprise entre ledit évidement central et ladite deuxième extrémité libre dudit lien contre une partie de ladite première face principale rejoignant la première desdites extrémités de ladite pièce de fixation auto-bloquante pour maintenir ledit lien en position fixe par rapport à ladite pièce de fixation auto-bloquante.

Ainsi, le lien entoure partiellement la pièce de fixation amovible de façon qu'une première portion soit disposée au regard de la première face principale, que le lien prenne appui sur la seconde extrémité de ladite pièce de fixation, qu'il traverse l'évidement central et qu'une deuxième portion débouchant dans ladite première face principale soit interposée entre ladite première face principale et ladite première portion de lien, parallèlement à lui. De la sorte, ladite première portion de lien qui prolonge directement une partie de lien qui entoure l'apophyse épineuse et qui est guidée sensiblement dans un plan comprenant ladite première face principale dans le prolongement de la première desdites extrémités de ladite pièce de fixation, est tendue par l'extension en translation de la seconde extrémité libre dudit lien qui prolonge la seconde portion de lien comprise entre la première portion de lien et la première face principale. Plus le lien est tendu plus ladite première portion de lien comprime la deuxième portion de lien contre la première face principale et la maintient en position fixe par apport à ladite pièce de fixation auto-bloquante amovible.

Selon une caractéristique particulièrement avantageuse, ladite pièce de fixation auto-bloquante amovible présente un évidement central formant fente dont le plan moyen forme un angle aigu avec ladite première partie de ladite première face principale de façon à former une arête dans chacune des faces principales susceptible de former des premiers moyens de frottement pour ledit lien. Ainsi, ledit lien est freiné en translation dans ladite pièce de fixation auto-bloquante, à la fois par l'angle que forme la paroi interne de l'évidement central et ladite première partie de première face principale, et par l'angle que forme la paroi interne de l'évidement central et une partie de la seconde face principale. En outre, de manière avantageuse, la seconde desdites extrémités de ladite pièce de fixation auto-bloquante amovible contre laquelle s'appuie ledit lien, présente deux faces inclinées l'une par rapport à l'autre de façon à former une arête susceptible de former des deuxièmes moyens de frottement pour ledit lien.

Selon un mode de réalisation préférentiel, lesdits deuxièmes moyens de liaison comprennent un logement ménagé dans ladite paroi latérale de ladite cale dont les deux bords opposés, sensiblement parallèles audit axe de la cale, présentent des moyens formant butée s'étendant sensiblement perpendiculairement à ladite paroi latérale de ladite cale, et lesdits premiers moyens de liaison font saillies dans les bords latéraux de ladite pièce de fixation auto-bloquante amovible pour venir en appui contre lesdits moyens formant butée de façon à bloquer ladite pièce de fixation auto-bloquante amovible en translation par rapport à ladite cale, dans une direction sensiblement parallèle audit axe longitudinal correspondant à ladite seconde direction de blocage dudit lien en translation par rapport à ladite cale.

Ainsi, la pièce de fixation auto-bloquante amovible est susceptible d'être encastrée au moins partiellement dans un logement et d'être immobilisée en translation selon une direction sensiblement parallèle à l'axe longitudinal de ladite cale, particulièrement selon ladite seconde direction correspondant au serrage dudit lien sur l'apophyse épineuse. Les premiers moyens de liaison faisant saillie dans les bords latéraux de ladite pièce de fixation, ils viennent en appui contre les moyens formant butée lorsque ledit lien est entraîné pour le serrage.

Préférentiellement, ledit logement présente une extrémité ouverte située au regard de ladite première desdites extrémités de la pièce de fixation auto-bloquante amovible à travers laquelle ledit lien est susceptible de coulisser. Ainsi, ledit lien est susceptible d'être guidé par les parois de ladite extrémité ouverte lors de sont déplacement en translation de façon à maintenir sensiblement ledit lien dans un plan comprenant ladite première face principale de façon à comprimer contre elle ladite deuxième portion de lien. La partie de lien disposée au regard de la première face principale se prolonge d'une part vers l'apophyse épineuse et d'autre part vers ladite seconde desdites extrémités pour l'entourer et s'appliquer contre ladite première partie de face principale après passage dans ledit évidement formant fente ; ledit lien formant une boucle dont les deux extrémités débouchent dans ladite extrémité ouverte.

Selon un mode préféré de mise en oeuvre de l'invention, lesdits moyens formant butée sont constitués par au moins deux évidements oblongs en regard pratiqués sensiblement perpendiculairement à ladite paroi latérale de la cale dans les deux bords opposés dudit logement ; et lesdits premiers moyens de liaison forment des tétons faisant saillie dans les bords latéraux de ladite pièce de fixation auto-bloquante, dans le prolongement l'un de l'autre, de façon à s'encastrer perpendiculairement à ladite paroi latérale dans lesdits évidements oblongs.

De la sorte, lorsque ledit lien est déplacé en translation pour serrer l'apophyse épineuse dans ladite gorge, la pièce de fixation auto-bloquante est entraînée en translation selon une direction parallèle à ladite seconde direction et lesdits tétons viennent en appui contre le bord des évidements oblongs de façon à bloquer ladite pièce. Avantageusement, lesdits évidements oblongs présentent une portion étranglée élastiquement déformable située entre une portion d'entrée et une portion de fond, susceptible de se déformer par enfoncement à force dudit téton de façon que ledit téton soit maintenu en position fixe dans ladite portion de fond. De la sorte, ladite pièce de fixation auto-bloquante amovible est rendue partiellement solidaire de ladite paroi latérale par une simple pression, ce qui facilite le montage.

Avantageusement, ledit implant comprend deux liens susceptibles d'entourer une apophyse épineuse supérieure et une apophyse épineuse inférieure, et deux pièces de fixation auto-bloquante amovibles susceptibles d'être reliées aux deux parois latérales de ladite cale. Ainsi, les apophyses sont reliées indépendamment les unes des autres à ladite cale.

Dans un mode de réalisation particulièrement avantageux, ladite cale et ladite pièce de fixation auto-bloquante amovible sont réalisées en matière plastique.

D'autres particularités et avantages de l'invention ressortiront à la lecture de la description faite ci-après de modes de réalisation particuliers de l'invention, donnés à titre indicatif mais non limitatif, en référence aux dessins annexés sur lesquels :
- la Figure 1 est une vue schématique éclatée de profil montrant l'implant conforme à l'invention ; et,
- la Figure 2, est une vue schématique en coupe verticale de l'invention illustrée sur la Figure 1, l'implant étant monté.

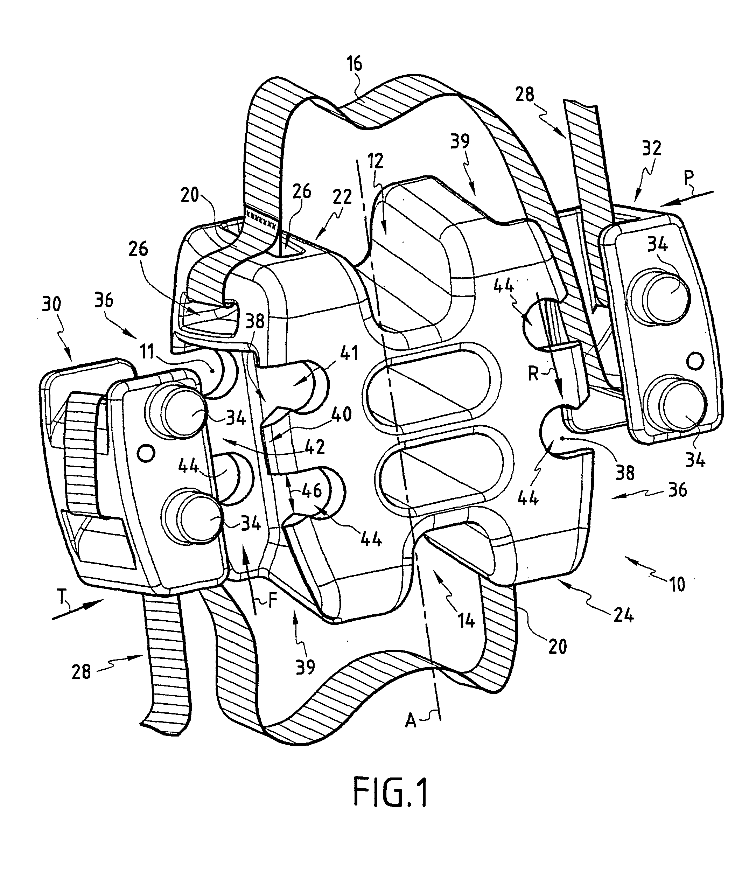

La Figure 1 représente un implant intervertébral comprenant une cale 10 présentant une gorge supérieure 12 et une gorge inférieure 14, les deux gorges 10 et 12 étant opposée l'une de l'autre et traversée par un axe longitudinal A. Chacune des gorges 12 et 14 sont susceptibles de recevoir une apophyse épineuse et la cale est montée entre les deux apophyses épineuses de deux vertèbres de façon à en limiter le déplacement l'une vers l'autre.

L'implant comporte en outre, deux liens formant bande, 16 et 18, les premières extrémités 20 de ces liens sont reliées, respectivement, à chacune des deux ailes 22 et 24 diagonalement opposées. La liaison entre la première extrémité 20 d'un lien et l'aile 22, 24, est réalisée au moyen d'une perçage 26 traversant l'extrémité de l'aile, 22, 24 et par la formation d'une boucle cousue de la première extrémité, traversant le perçage. Les premières extrémités 20 des liens 16 et 18 sont montées de façon symétrique sur les ailes 22 et 24, généralement, avant l'intervention chirurgicale.

Par ailleurs, une caractéristique de l'implant réside dans le mode d'accrochage de la seconde extrémité des liens 28 au moyen de deux pièces de fixation auto-bloquante amovibles 30, 32 à travers lesquelles les liens respectivement 18 et 16 sont susceptibles de coulisser selon des directions F et R. En revanche, comme on l'expliquera plus en détails dans la suite de la description, les liens 18 et 16 sont bloqués en translation dans les directions opposées à F et à R lorsqu'ils sont en position tendue.

Les pièces de fixation auto-bloquante amovibles 30, 32, outre le fait qu'elles permettent l'accrochage des liens 16, 18 sur la cale 10, laissent la possibilité d'être reliées à la cale 10 après que cette dernière a été installée dans l'espace intervertébrale et que les liens 16 et 18 ont été insérés dans les pièces de fixation auto-bloquante amovibles 30, 32.

A cet effet, les pièces de fixation auto-bloquante amovibles 30, 32 présentent des premiers moyens de liaison 34 formant tétons et faisant saillie dans les bords latéraux des pièces de fixation 30, 32. La Figure 1 illustre principalement une face de l'implant intervertébral, l'autre face, symétrique par rapport à un plan vertical n'apparaît pas sur la Figure 1. Ainsi, les tétons 34 ont respectivement leur symétrique dans les bords latéraux des pièces de fixation 30, 32. En outre, les parois latérales 36 de la cale 10 présentent des deuxièmes moyens de liaison 38 formant un logement dont au moins une extrémité 39 est ouverte et dont les deux bords opposés 40 et 42 parallèles entre eux et à l'axe A de la cale 10 présentent des évidements oblongs 44 formant butées. Bien entendu, la largeur du logement 38 séparant les deux bords opposés 40, 42 est supérieure à la largeur des pièces de fixation 30, 32 séparant les deux bords latéraux opposés de façon que les pièces de fixation 30, 32 soient insérées au moins partiellement dans lesdits logements 38.

Les bords opposés 40 et 42 du logement 38 présentent deux paires d'évidements oblongs en regard susceptibles de coopérer avec les tétons 34 faisant saillie dans les bords latéraux des pièces de fixation 30, 32. Ainsi, la distance qui sépare les deux évidements oblongs d'un bord 40 ou 42 est sensiblement équivalente à la distance qui sépare deux tétons 34 d'un bord latéral d'une pièce de fixation 30 ou 32.

En outre, les évidements oblongs 44 présentent un étranglement 46, déformable, situé entre l'entrée de l'évidement oblong 44 et le fond de l'évidement 44, de façon que les tétons 34 dont le diamètre est sensiblement supérieur à la largeur de l'étranglement 46 puissent être enfoncés à force dans l'évidement oblong 44. Bien évidemment, la largeur du fond des évidements oblongs correspond au diamètre des tétons 44.

La cale 10 et les pièces de fixation 30, 32 sont réalisées en matière plastique et présentent un module d'élasticité suffisamment bas pour que, à la fois l'étranglement 46 et le téton 34 se déforment élastiquement et que le téton soit inséré dans le fond de l'évidement oblong 44. Le raisonnement tenu pour la coopération d'un téton 34 avec un évidement oblong 44 est bien entendu susceptible d'être tenu pour tous les tétons 34 et tous les évidements oblongs 44. Ainsi, les pièces de fixation auto-bloquante amovibles 30 et 32 sont encastrables dans leur logement respectif 38 selon la direction T pour la pièce 30 et selon la direction P pour la pièce 32. Lorsque les pièces 30, 32 sont encastrées dans des parois latérales de ladite cale 10, les quatre tétons 34 sont situés dans le fond des quatre évidements oblongs 44, et elles ne peuvent être retirées qu'en exerçant une traction sur les pièces 30 ou 32 et sur la cale 10 pour déformer à nouveau l'étranglement 46.

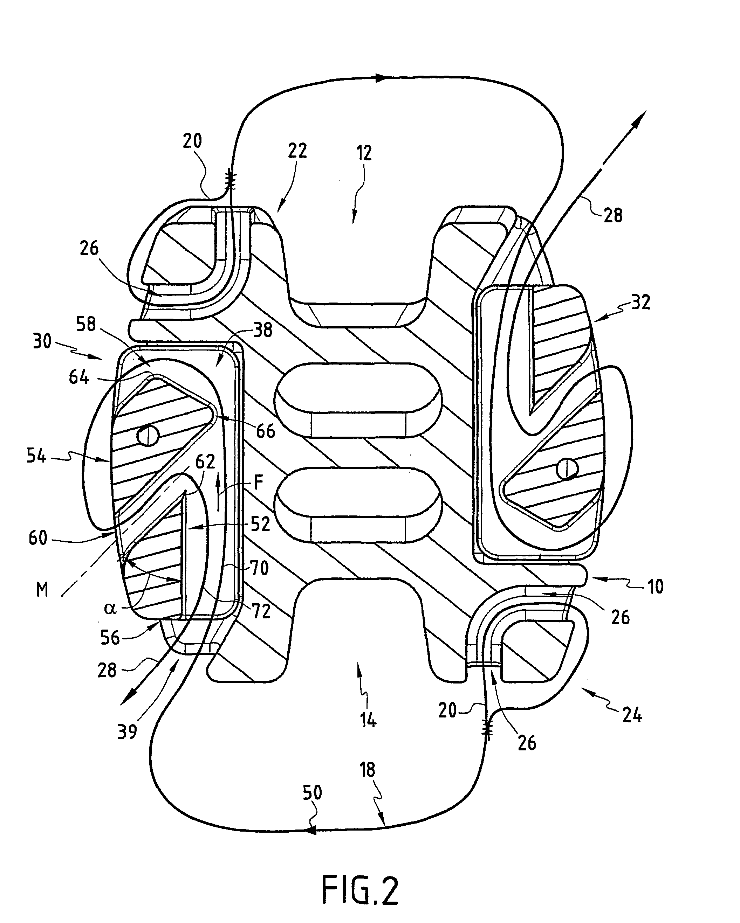

La Figure 2 illustre l'implant intervertébral lorsque les deux pièces de fixation 30 et 32 sont encastrées dans leur logement 38. En se référant désormais à cette Figure 2 et également à la Figure 1 on décrira les éléments constitutifs des pièces de fixation auto-bloquante permettant de bloquer les liens 16 et 18 en translation.

La description qui suit se référera à la pièce de fixation auto-bloquante amovible 30, mais elle s'applique de manière identique à la pièce de fixation 32.

On retrouve sur la Figure 2, le lien 18 dont la première extrémité 20 est reliée à l'aile 24, et dont une partie 50 est disposée au regard de la gorge 14 dans laquelle une apophyse épineuse, non représentée, est susceptible de venir en appui. Le lien 18 se prolonge ensuite vers l'autre aile de la gorge 14 pour rejoindre la pièce de fixation auto-bloquante 30. Comme on l'a expliqué dans la description qui précède, le lien 18 est inséré dans la pièce de fixation 30 préalablement à son encastrement dans le logement 38.

La pièce de fixation auto-bloquante 30 présente une première face principale 52 et une seconde face principale 54 opposée à la première, les deux faces opposées 52, 54 se rejoignant dans la première extrémité 56 et dans la seconde extrémité 58 de la pièce de fixation 30. Elle présente également un évidement central 60 formant fente débouchant dans la première face principale 52 et dans la seconde face principale 54. Le plan moyen M de l'évidement 60 forme un angle aigu alpha avec la première partie de la face principale, par exemple 30° de façon à former une arête 62 . En outre, la seconde extrémité 58 de la pièce de fixation 30 présente deux faces inclinées l'une par rapport à l'autre, formant une arête 64. De plus l'évidement 60 débouche au niveau de la jonction entre la première face principale et la seconde extrémité 58, formant ainsi une autre arête 66.

Les éléments précédemment décrits permettent de maintenir immobile en translation le lien 18 par rapport à ladite pièce de fixation 30 et par conséquent par rapport à la cale 10 puisque la pièce de fixation 30 est bloquée dans le logement 38.

Pour ce faire, et préalablement à l'encastrement des pièces de fixation auto-bloquante amovibles dans les parois latérales de la cale 10, l'extrémité libre 28 du lien 18 par exemple après avoir été introduit dans l'espace interépineux inférieur derrière le ligament interépineux, est inséré, en dehors du corps, dans l'évidement central 60 formant fente depuis la seconde face principale 54 de la pièce de fixation 30. Ensuite, la pièce de fixation 30 est portée au regard de la paroi latérale de la cale 10 en la faisant coulisser le long du lien 18, et en la disposant de façon que la première face principale soit au regard de la paroi latérale et, que la partie de lien 18 prolongeant l'espace interépineux inférieur et la partie de lien 18 se prolongeant par la seconde extrémité libre 28 débouchent dans l'extrémité ouverte 39 de la pièce de fixation 30. Ainsi, une partie de lien 18 prolongeant l'espace interépineux inférieur est appliqué contre la première face principale de la pièce de fixation 30, puis contourne la seconde extrémité 58 et s'applique contre une portion de la seconde face principale 54 avant de traverser l'évidement central 60 formant fente, pour déboucher ensuite dans la première face principale 52 et s'appuyer contre une partie de la première face principale 52 qui rejoint la première extrémité 56.

De la sorte, lorsqu'une traction est exercée sur la seconde extrémité libre 28 du lien 18 afin de le tendre et de solidariser l'apophyse épineuse et la cale 10, une première portion 70 de lien 18, comprime une deuxième portion 72 de lien 18 contre la première face principale et produit des forces de frottement aptes à immobiliser les portions 70 et 72 de lien 18 les unes par rapport aux autres. Sur la Figure 2, le lien 18 est illustré par un trait pour ne pas surcharger le dessin, mais le lien 18 présente une épaisseur telle que le contact entre les deux portions 70 et 72 est rendu possible avec la configuration de la pièce de fixation 30 et la cale 10.

Plus le lien 18 est tendu dans la pièce de fixation 30 et plus, non seulement les deux portions 70 et 72 de lien 18 sont comprimées ensemble, mais aussi les moyens de frottement formés par les arêtes 62 ou 66, par exemple, sont efficaces pour bloquer le lien en translation selon une direction opposée à F. De la sorte, le lien 18 est maintenu immobile en translation par rapport à la cale 10 et maintient la cale 10 sur l'apophyse épineuse.

Bien évidemment, de façon totalement symétrique la cale 10 est maintenue contre l'autre apophyse épineuse, au moyen du lien 16 et de la pièce de fixation 32.

## Revendications

1. Implant intervertébral comprenant une cale (10) dans laquelle sont ménagées deux gorges (12, 14) opposées l'une de l'autre que l'axe longitudinal (A) de ladite cale (10) traverse et qui sont susceptibles de recevoir les deux apophyses épineuses de deux vertèbres, ladite cale (10) présentant deux parois latérales et au moins un lien (16, 18) de fixation pour maintenir lesdites apophyses épineuses dans lesdites gorges (12, 14), la première extrémité (20) dudit lien (16, 18) étant susceptible d'être relié à ladite cale (10), ledit lien (16, 18) étant susceptible d'entourer au moins une apophyse épineuse et la seconde extrémité (28) dudit lien étant libre,
**caractérisé en ce qu'**il comprend au moins une pièce de fixation auto-bloquante amovible (30, 32) présentant des premiers moyens de liaison (34) et à travers laquelle ledit lien (16, 18) est susceptible de coulisser lorsqu'il est entraîné en translation selon une première direction (R, F), ladite pièce de fixation auto-bloquante (30, 32) étant susceptible de bloquer ledit lien (16, 18) en translation selon une seconde direction opposée à ladite première direction (R, F) ;
et **en ce que** au moins une des deux parois latérales (36) de ladite cale (10) comporte des deuxièmes moyens de liaison (38) aptes à coopérer avec lesdits premiers moyens de liaison (34) pour relier ladite pièce de fixation auto-bloquante amovible (30, 32) à la paroi latérale (36) de ladite cale (10), l'entraînement de ladite extrémité libre (28) dudit lien (16, 18) de façon à entraîner ledit lien (16, 18) en translation selon ladite première direction (R, F) produisant le serrage de ladite apophyse épineuse dans ladite gorge (12, 14) et le blocage dudit lien (16, 18) en translation selon ladite seconde direction par rapport à ladite cale (10).

2. Implant intervertébral selon la revendication 1, **caractérisé en ce que** ladite pièce de fixation auto-bloquante amovible (30, 32) présente une première face principale (52) opposée à une seconde face principale (54) qui sont reliées aux deux extrémités (56, 58) de ladite pièce de fixation auto-bloquante amovible (30, 32) et un évidement central (60) formant fente, débouchant dans lesdites faces principales (52, 54), que ledit lien (16, 18) traverse, de façon qu'une première portion (70) dudit lien (16, 18) comprise entre ladite première extrémité (20) et ledit évidement central (60) soit susceptible de comprimer une deuxième portion (72) dudit lien comprise entre ledit évidement central (60) et ladite deuxième extrémité libre (28) dudit lien contre une partie de ladite première face principale (52) rejoignant la première (56) desdites extrémités de ladite pièce de fixation auto-bloquante (30, 32) pour maintenir ledit lien (16, 18) en position fixe par rapport à ladite pièce de fixation auto-bloquante (30, 32).

3. Implant intervertébral selon la revendication 2, **caractérisé en ce que** ladite pièce de fixation auto-bloquante amovible (30, 32) présente un évidement central formant fente (60) dont le plan moyen (M) forme un angle aigu (α) avec ladite première partie de ladite première face principale (52) de façon à former une arête (62) dans chacune des faces principales (52, 54) susceptible de former des premiers moyens de frottement pour ledit lien (16, 18).

4. Implant intervertébral selon la revendication 2 ou 3, **caractérisé en ce que** la seconde (58) desdites extrémités de ladite pièce de fixation auto-bloquante amovible (30, 32) contre laquelle s'appuie ledit lien (16, 18), présente deux faces inclinées l'une par rapport à l'autre de façon à former une arête (64) susceptible de former des deuxièmes moyens de frottement pour ledit lien.

5. Implant intervertébral selon l'une quelconque des revendications 1 à 4,
**caractérisé en ce que** lesdits deuxièmes moyens de liaison (38) comprennent un logement ménagé dans ladite paroi latérale (36) de ladite cale (10) dont les deux bords opposés (40, 42), sensiblement parallèles audit axe (A) de la cale, présentent des moyens (44) formant butée s'étendant sensiblement perpendiculairement à ladite paroi latérale (36) de ladite cale,
et **en ce que** lesdits premiers moyens de liaison (34) font saillie dans les bords latéraux de ladite pièce de fixation auto-bloquante amovible (30, 32) pour venir en appui contre lesdits moyens (44) formant butée de façon à bloquer ladite pièce de fixation auto-bloquante amovible (30, 32) en translation par rapport à ladite cale, dans une direction sensiblement parallèle audit axe longitudinal (A) correspondant à ladite seconde direction de blocage dudit lien en translation par rapport à ladite cale (10).

6. Implant intervertébral selon la revendication 5, **caractérisé en ce que** ledit logement présente une extrémité ouverte (39) située au regard de ladite première (56) desdites extrémités de la pièce de fixation auto-bloquante amovible (30, 32) à travers laquelle ledit lien (16, 18) est susceptible de coulisser.

7. Implant intervertébral selon la revendication 5 ou 6,
**caractérisé en ce que** lesdits moyens (44) formant butée sont constitués par au moins deux évidements oblongs en regard pratiqués sensiblement perpendiculairement à ladite paroi latérale (36) de la cale dans les deux bords opposés (40, 42) dudit logement ;
et **en ce que** lesdits premiers moyens de liaison (34) forment des tétons faisant saillie dans les bords latéraux de ladite pièce de fixation auto-bloquante (30, 32), dans le prolongement l'un de l'autre, de façon à s'encastrer perpendiculairement à ladite paroi latérale (36) dans lesdits évidements oblongs (44).

8. Implant intervertébral selon la revendication 7, **caractérisé en ce que** lesdits évidements oblongs (44) présentent une portion étranglée (46) élastiquement déformable située entre une portion d'entrée et une portion de fond, susceptible de se déformer par enfoncement à force dudit téton (34) de façon que ledit téton (34) soit maintenu en position fixe dans ladite portion de fond.

9. Implant intervertébral selon l'une quelconque des revendications 1 à 8, **caractérisé en ce qu'**il comprend deux liens (16, 18) susceptibles d'entourer respectivement une apophyse épineuse supérieure et une apophyse épineuse inférieure, et deux pièces de fixation auto-bloquante amovibles (30, 32) susceptibles d'être reliées aux deux parois latérales (36) de ladite cale (10).

10. Implant intervertébral selon l'une quelconque des revendications 1 à 9, **caractérisé en ce que** ladite cale (10) et ladite pièce de fixation auto-bloquante amovible (30, 32) sont réalisés en matière plastique.

## Patentansprüche

1. Zwischenwirbelimplantat mit einem Keil (10), in dem zwei Hohlkehlen (12, 14) einander gegenüberliegend eingerichtet sind, welche von der Längsachse (A) des Keils (10) durchquert werden, und die zwei Gelenkfortsätze von zwei Wirbeln aufnehmen können, wobei der Keil (10) zwei seitliche Wände und mindestens eine Verbindung (16, 18) zum Befestigen umfasst, um die Gelenkfortsätze in den Hohlkehlen (12, 14) zu halten, wobei das erste Ende (20) der Verbindung (16, 18) mit dem Keil (10) verbunden werden kann, wobei die Verbindung (16, 18) mindestens einen Gelenkfortsatz umgeben kann und das zweite Ende (28) der Verbindung frei ist,
**dadurch gekennzeichnet, dass** es mindestens ein abnehmbares selbstblockierendes Befestigungsteil (30, 32) umfasst, das erste Verbindungsmittel (34) aufweist, und durch welches die Verbindung (16, 18) gleiten kann, wenn sie in Translation in einer ersten Richtung (R, F) angetrieben wird, wobei das selbstblockierende Befestigungsteil (30, 32) die Verbindung (16, 18) in Translation in einer zweiten Richtung, die der ersten Richtung (R, F) entgegengesetzt ist, blockieren kann;
und dadurch, dass mindestens eine der zwei Seitenwände (36) des Keils (10) zweite Verbindungsmittel (38) umfasst, die mit den ersten Verbindungsmitteln (34) zusammenwirken können, um das abnehmbare selbstblockierende Befestigungsteil (30, 32) mit der Seitenwand (36) des Keils (10) zu verbinden, wobei das Antreiben des freien Endes (28) der Verbindung (16, 18) das Antreiben der Verbindung (16, 18) in Translation in der ersten Richtung (R, F), das Spannen des Gelenkfortsatzes in der Hohlkehle (12, 14) und das Blockieren der Verbindung (16, 18) in Translation in der zweiten Richtung zum Keil (10) bewirkt.

2. Zwischenwirbelimplantat nach Anspruch 1, **dadurch gekennzeichnet, dass** das abnehmbare selbstblockierende Befestigungsteil (30, 32) eine erste Hauptfläche (52), die einer zweiten Hauptfläche (54) gegenüber liegt, welche Hauptflächen mit den zwei Enden (56, 58) des abnehmbaren selbstblockierenden Befestigungsteils (30, 32) verbunden sind, und eine zentrale Aussparung (60) aufweist, die einen Schlitz bildet, der in die Hauptflächen (52, 54), welche von der Verbindung (16, 18) durchquert werden, mündet, so dass ein erster Teil (70) der Verbindung (16, 18), der zwischen dem ersten Ende (20) und der zentralen Aussparung (60) enthalten ist, einen zweiten Teil (72) der Verbindung zwischen der zentralen Aussparung (60) und dem zweiten freien Ende (28) der Verbindung gegen einen Teil der ersten Hauptfläche (52) komprimieren kann, die auf das erste (56) der Enden des selbstblockierenden Befestigungsteils (30, 32) trifft, um die Verbindung (16, 18) zu dem selbstblockierenden Befestigungsteil (30, 32) in stationärer Stellung zu halten.

3. Zwischenwirbelimplantat nach Anspruch 2, **dadurch gekennzeichnet, dass** das abnehmbare selbstblockierende Befestigungsteil (30, 32) eine zentrale Aussparung (60) umfasst, die einen Schlitz bildet, dessen mittlere Ebene (M) einen spitzen Winkel (α) mit dem ersten Teil der ersten Hauptfläche (52) bildet, so dass eine Kante (62) in jeder der Hauptflächen (52, 54) entsteht, die erste Reibungsmittel für die Verbindung (16, 18) bilden kann.

4. Zwischenwirbelimplantat nach Anspruch 2 oder 3, **dadurch gekennzeichnet, dass** das zweite (58) der Enden des abnehmbaren selbstblockierenden Befestigungsteils (30, 32), an welchem die Verbindung (16, 18) aufliegt, zwei Flächen aufweist, die zueinander so geneigt sind, dass eine Kante (64) entsteht, die zweite Reibungsmittel für die Verbindung bilden kann.

5. Zwischenwirbelimplantat nach einem der Ansprüche 1 bis 4,
**dadurch gekennzeichnet, dass** die zweiten Verbindungsmittel (38) eine Aufnahme umfassen, die in der Seitenwand (36) des Keils (10) eingerichtet ist, deren gegenüberliegende Ränder (40, 42), die im Wesentlichen zur Achse (A) des Keils parallel sind, Mittel (44) aufweisen, die einen Anschlag bilden, der sich im Wesentlichen senkrecht zur Seitenwand (36) des Keils (10) erstreckt.
und dadurch, dass die ersten Verbindungsmittel (34) aus den Seitenrändem des abnehmbaren selbstblockierenden Befestigungsteils (30, 32) vorstehen, um gegen die Mittel (44) zum Aufliegen kommen, die den Anschlag bilden, so dass das abnehmbare selbstblockierende Befestigungsteil (30, 32) in Translation zum Keil (10) in eine im Wesentlichen zur Längsachse (A) parallele Richtung, die der zweiten Blockierungsrichtung der Verbindung in Translation zum Keil (10) entspricht, blockiert wird.

6. Zwischenwirbelimplantat nach Anspruch 5, **dadurch gekennzeichnet, dass** die Aufnahme ein offenes Ende (39) umfasst, die gegenüber dem ersten (56) der Enden des abnehmbaren selbstblockierenden Befestigungsteils (30, 32) liegt, durch welches die Verbindung (16, 18) gleiten kann.

7. Zwischenwirbelimplantat nach Anspruch 5 oder 6,
**dadurch gekennzeichnet, dass** die Mittel (44), die den Anschlag bilden, aus mindestens zwei länglichen Aussparungen einander gegenüber liegend bestehen, die im Wesentlichen senkrecht zur Seitenwand (36) des Keils in die zwei gegenüberliegenden Ränder (40, 42) der Aufnahme gearbeitet sind;
und dadurch, dass die ersten Verbindungsmittel (34) Zapfen bilden, die aus den seitlichen Ränder des selbstblockierenden Befestigungsteils (30, 32) in Verlängerung zueinander vorstehen, so dass sie sich senkrecht zur Seitenwand (36) in die länglichen Aussparungen (44) einlassen.

8. Zwischenwirbelimplantat nach Anspruch 7, **dadurch gekennzeichnet, dass** die länglichen Aussparungen (44) einen eingeschnürten Teil (46) aufweisen, der elastisch verformbar ist und zwischen einem Eingangsteil und einem Bodenteil liegt, der sich durch Eindrücken unter Kraft des Zapfens (34) verformen kann, so dass der Zapfen (34) im Bodenteil in stationärer Stellung gehalten wird.

9. Zwischenwirbelimplantat nach einem der Ansprüche 1 bis 8, **dadurch gekennzeichnet, dass** es zwei Verbindungen (16, 18) umfasst, die jeweils einen oberen und einen unteren Gelenkfortsatz umgeben können, und zwei abnehmbare selbstblockierende Befestigungsteile (30, 32), die mit den Seitenwänden (36) des Keils (10) verbunden werden können.

10. Zwischenwirbelimplantat nach einem der Ansprüche 1 bis 9, **dadurch gekennzeichnet, dass** der Keil (10) und das abnehmbare selbstblockierende Befestigungsteil (30, 32) aus Kunststoff hergestellt sind.

## Claims

1. An intervertebral implant comprising a block (10) with grooves (12, 14) on two opposite sides and on a longitudinal axis (A) of said block (10) adapted to receive the spinous processes of two vertebrae, said block (10) having two lateral walls and at least a fixing tie (16, 18) for retaining said spinous processes in said grooves (12, 14), a first end (20) of said tie (16, 18) being adapted to be connected to said block (10), said tie (16, 18) being adapted to surround at least one spinous process, and the second end (28) of said tie being a free end,
which implant is **characterised in that** it includes at least a removable self-locking fixing member (30, 32) having first connecting means (34) and through which said tie (16, 18) can slide when it moves in translation in a first direction (R, F), said self-locking fixing member (30, 32) being adapted to immobilize said tie (16, 18) against movement in translation in a second direction opposite said first direction (R, F);
and **in that** at least one of two lateral walls (36) of said block (10) includes second connecting means (38) adapted to cooperate with said first connecting means (34) to connect said removable self-locking fixing member (30, 32) to the lateral wall (36) of said block (10), movement of said free end (28) of said tie (16, 18) to move said tie (16, 18) in translation in said first direction (R, F) causing said spinous process to be clamped in said groove (12, 14) and said tie (16, 18) to be immobilized against movement in translation relative to said block (10) in said second direction.

2. An intervertebral implant according to claim 1, **characterised in that** said removable self-locking fixing member (30, 32) has a first main face (52) opposite a second main face (54), which main faces are joined together at two ends (56, 58) of said removable self-locking fixing member (30, 32) and a central slot (60) opening into said main faces (52, 54) and through which said tie (16, 18) passes, so that a first portion (70) of said tie (16, 18) between said first end (20) and said central slot (60) can press a second portion (72) of said tie between said central opening (60) and said free second end (28) of said tie against a portion of said first main face (52) adjoining the first end (56) of said self-locking fixing member (30, 32) to retain said tie (16, 18) in a fixed position relative to said self-locking fixing member (30, 32).

3. An intervertebral implant according to claim 2, **characterised in that** said removable self-locking fixing member (30, 32) has a central slot (60) whose median plane (M) is at an acute angle (α) to said first portion of said first main face (52) to form an edge (62) in each of the main faces (52, 54) adapted to form first friction means for said tie (16, 18).

4. An intervertebral implant according to claim 2 or claim 3, **characterised in that** the second end (58) of said ends of said removable self-locking fixing member (30, 32) against which said tie (16, 18) bears has two faces inclined to each other to form an edge (64) adapted to form second friction means for said tie.

5. An intervertebral implant according to any one of claims 1 to 4,
**characterised in that** said second connecting means (38) include a housing in said lateral wall (36) of said block (10) whose two opposite edges (40, 42), which are substantially parallel to said axis (A) of the block, include abutment means (44) substantially perpendicular to said lateral wall (36) of said block,
and **in that** said first connecting means (34) project from lateral sides of said removable self-locking fixing member (30, 32) to bear against said abutment means (44) to immobilize said removable self-locking fixing member (30, 32) against movement in translation relative to said block in a direction substantially parallel to said longitudinal axis (A) and corresponding to said second direction of immobilization of said tie against movement in translation relative to said block (10).

6. An intervertebral implant according to claim 5, **characterised in that** said housing has an open end (39) facing said first end (56) of the removable self-locking fixing member (30, 32) and through which said tie (16, 18) can slide.

7. An intervertebral implant according to claim 5 or claim 6,
**characterised in that** said abutment means (44) consist of at least two facing oblong openings substantially perpendicular to said lateral wall (36) of the block in two opposite edges (40, 42) of said housing;
and **in that** said first connecting means (34) consist of studs projecting from the lateral sides of said self-locking fixing member (30, 32) and aligned with each other, so that they can be inserted into said oblong openings (44) perpendicularly to said lateral wall (36).

8. An intervertebral implant according to claim 7, **characterised in that** said oblong openings (44) have an elastically deformable constricted portion (46) between an entry portion and a closed end portion and adapted to be deformed by forcibly pressing in said stud (34) so that said stud (34) is held fixed in position in said closed end portion.

9. An intervertebral implant according to any one of claims 1 to 8, **characterised in that** it includes two ties (16, 18) respectively adapted to surround an upper spinous process and a lower spinous process and two removable self-locking fixing members (30, 32) adapted to be connected to two lateral walls (36) of said block (10).

10. An intervertebral implant according to any one of claims 1 to 9, **characterised in that** said block (10) and said removable self-locking fixing member (30, 32) are made of plastics materials.
